(19) Europäisches Patentamt
European Patent Office
Office européen des brevets

(11) EP 1 678 305 B1

(12) **FASCICULE DE BREVET EUROPEEN**

(45) Date de publication et mention de la délivrance du brevet:
**23.11.2011 Bulletin 2011/47**

(21) Numéro de dépôt: **04805294.8**

(22) Date de dépôt: **25.10.2004**

(51) Int Cl.:
***C12N 15/30*** *(2006.01)*

(86) Numéro de dépôt international:
**PCT/FR2004/002735**

(87) Numéro de publication internationale:
**WO 2005/044844 (19.05.2005 Gazette 2005/20)**

# (54) SOUCHE DE PROTOZOAIRE DE L'ESPÈCE LEISHMANIA INFANTUM DE VIRULENCE ATTENUEE ET SON UTILISATION

PROTOZOANSTAMM DER SPEZIES LEISHMANIA INFANTUM MIT REDUZIERTER VIRULENZ UND DESSEN VERWENDUNG

PROTOZOAN STRAIN OF THE SPECIES LEISHMANIA INFANTUM OF REDUCED VIRULENCE AND USE THEREOF

(84) Etats contractants désignés:
**AT BE BG CH CY CZ DE DK EE ES FI FR GB GR HU IE IT LI LU MC NL PL PT RO SE SI SK TR**

(30) Priorité: **29.10.2003 FR 0312689**

(43) Date de publication de la demande:
**12.07.2006 Bulletin 2006/28**

(73) Titulaires:
- **Institut de Recherche pour le Développement ( IRD)**
  **75480 Paris Cedex 10 (FR)**
- **Universidade Do Porto**
  **4050 345 Porto (PT)**

(72) Inventeurs:
- **CORDEIRA DA SILVA, Anabela**
  **P-4250 Porto (PT)**
- **OUAISSI, Ali**
  **F-34150 La Boissière (FR)**
- **SERENO, Denis**
  **F-34560 Poussan (FR)**
- **VERGNES, Jean-Baptiste**
  **F-23500 Felletin (FR)**

(74) Mandataire: **Grosset-Fournier, Chantal Catherine**
**Grosset-Fournier & Demachy**
**54, rue Saint-Lazare**
**75009 Paris (FR)**

(56) Documents cités:
- **CORDEIRO-DA-SILVA A ET AL: "Identification of antibodies to Leishmania silent information regulatory 2 ( SIR2 ) protein homologue during canine natural infections: pathological implications." IMMUNOLOGY LETTERS, (2003 APR 3) 86 (2) 155-62., avril 2003 (2003-04), XP001181346**
- **TITUS R G ET AL: "DEVELOPMENT OF A SAFE LIVE LEISHMANIA VACCINE LINE BY GENE REPLACEMENT" PROCEEDINGS OF THE NATIONAL ACADEMY OF SCIENCES OF USA, NATIONAL ACADEMY OF SCIENCE. WASHINGTON, US, vol. 92, octobre 1995 (1995-10), pages 10267-10271, XP002052134 ISSN: 0027-8424 cité dans la demande**
- **GARCIA-SALCEDO JOSE A ET AL: "A chromosomal SIR2 homologue with both histone NAD-dependent ADP-ribosyltransferase and deacetylase activities is involved in DNA repair in Trypanosoma brucei." EMBO JOURNAL, (2003 NOV 3) 22 (21) 5851-62., novembre 2003 (2003-11), XP002281455**
- **VERGNES BAPTISTE ET AL: "Cytoplasmic SIR2 homologue overexpression promotes survival of Leishmania parasites by preventing programmed cell death." GENE, (2002 AUG 21) 296 (1-2) 139-50., août 2002 (2002-08), XP004386950 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 678 305 B1

## Description

La présente invention a pour objet des souches de protozoaires de virulence atténuée et leur utilisation.

Les protozoaires flagellés de la famille des *Trypanosomatidae* sont responsables de nombreuses maladies affectant l'homme et l'animal.

Ainsi, parmi les protozoaires du genre *Trypanosoma, T. brucei* et *T. cruzi* sont par exemple respectivement responsable de la maladie du sommeil et de la maladie de Chagas.

Les protozoaires du genre *Leishmania,* tels que *L. aethiopica, L donovani, L. infantum, L. major, L. mexicana* ou *L. tropica* sont eux responsables des leishmanioses. Ces infections sont endémiques dans plus de 88 pays et touchent une portion notable du territoire français. Sur le pourtour méditerranéen, et en France en particulier, les leishmanioses sont essentiellement le fait de *L. infantum.* Dans le monde, l'OMS estime à 12 millions le nombre d'individus parasités et plus de 350 millions de personnes seraient quotidiennement exposées. Il existe trois formes majeures de leishmaniose dont la forme la plus sévère, la leishmaniose viscérale, peut s'avérer mortelle en absence de traitement. Cette situation est aggravée, depuis l'émergence du VIH, par le fait qu'on rencontre de plus en plus ces infections en tant que maladies opportunistes chez les personnes atteintes du syndrome d'immunodéficience acquise (SIDA), notamment dans le Sud-Ouest de l'Europe. Le parasite utilise le statut immunitaire déprimé de son hôte pour s'implanter ou se réactiver.

Les traitements actuels des leishmanioses font appels à des médicaments de maniement difficile, tels que l'amphotéricine B ou des médicaments appartenant à la famille des antimoniés, dont les effets secondaires sont importants.

A ce jour, il n'existe pas de vaccins permettant de prévenir les infections à *Leishmania.* La mise au point de tels vaccins fait l'objet de nombreux programmes de recherche. Trois axes principaux sont privilégiés ; la préparation de vaccins tués, de vaccins vivants atténués ou de vaccins sous-unitaires. Parmi ces trois axes, la préparation de vaccins vivants atténués semble être la plus prometteuse ; ce type de vaccin permet en effet une immunisation la plus proche de celle réalisée par le parasite sauvage. De plus, les techniques modernes de biologie moléculaire permettent de préparer des souches de virulence atténuée par inactivation spécifique de certains gènes de virulence. Plusieurs souches de *Leishmania* dont un gène de virulence a été inactivé ont ainsi été préparées (Titus et al. (1995) Proc. Natl. Acad Sci. USA, 92:10267-10271, WO 98/44943, EP 0 716 697, EP 0 806 476). Le choix du gène à inactiver est crucial car il conditionne l'obtention d'une souche adéquatement atténuée, c'est-à-dire ayant une capacité réplicative suffisante pour stimuler le système immunitaire et cependant limitée afin d'éviter tout risque infectieux, en particulier chez le sujet immunodéprimé.

Les protéines SIR2, découvertes à l'origine chez la levure, sont connues pour intervenir dans la condensation de la chromatine par désacétylation des histones au niveau de certaines régions chromosomiques : les loci *HMR* et *HML* responsables de la commutation de type sexuel, les régions sub-télomériques, et les répétitions d'ADNr où SIR2 prévient la formation de minicercles extra-chromosomiques par recombinaison homologue (Guarente et al. (1999) Nat. Genet. 23:281-5 ; Gartenberg et al. (2000) Curr. Opin. Microbiol. 3:132-7 ; Min et al. (2001) Cell 105:269-79). D'autres travaux ont démontré son implication dans les mécanisme de réparation d'ADN (Tsukamoto et al. (1997) Nature 388: 900-3).

Le rôle biologique de SIR2 n'est cependant pas restreint au « silencing » et à la réparation de l'ADN, et la fonction la plus étonnante attribuée à SIR2 est certainement d'augmenter la longévité des organismes possédant une copie supplémentaire de ce gène. Ceci a été démontré chez la levure et *Caenorhabditis elegans* (Guarente et al. (2000) Genes Dev. 14:1021-6 ; Tissenbaum et al. (2001) Nature 410:227-30).

Les programmes de séquençage systématique ont permis de caractériser d'autres protéines apparentées à SIR2 chez différents organismes eucaryotes et procaryotes. On parle désormais de famille protéique comprenant les protéines SIR2 nucléaires proprement dites et les homologues de SIR2 indifféremment appelées « Hst » pour « Homologue of SIR Two » ou « sirtuin » (Frye et al. (2000) Biochem. Biophys. Res. Commun. 273:793-8). La signification biologique de cette diversité des protéines SIR2 n'est pas encore clairement établie, mais différents travaux montrent que toutes les Hst étudiées possèdent au même titre que SIR2 une activité enzymatique de type histone désacétylase qui est strictement dépendante du NAD. Cette particularité a permis de regrouper les protéines SIR2 dans une nouvelle classe d'histone désacétylase dite de classe III en opposition aux classes I et II déjà caractérisées.

L'existence d'homologues de SIR2 ayant des localisations strictement cytoplasmiques, et la présence d'Hst chez les procaryotes qui ne possèdent pas d'histones, suppose cependant que ces enzymes possèdent d'autres substrats physiologiques que les histones et donc d'autres fonctions biologiques. Les récents travaux publiés dans ce domaine montrent effectivement un nombre croissant de « cibles » caractérisées de SIR2 ou de ses homologues. SIRT1 chez l'homme et son homologue murin sont ainsi capables de se fixer spécifiquement à p53 et de l'inactiver par désacétylation (Vaziri et al. (2001) Cell 107:149-59 ; Luo et al. (2001) Cell 107:137-48). SIRT1, par ce biais, prévient l'entrée en apoptose de cellules soumises à un stress. Le facteur de transcription TAF168 chez la souris (Muth et al. (2001) Embo J. 20:1353-62), la protéine Alba chez l'archéobactérie *S. solfataricus* (Bell et al. (2002) Science 296: 148-51) ou plus récemment le proto-oncogène BCL6 chez l'homme (Bereshchenko et al. (2002) Nat. Genet. 32:606-13) sont tous désacétylés par SIR2 ou ses homologues. Cette diversité de partenaires sans interrelation directe et le nombre de processus biologiques dans lesquels elles sont impliquées dénotent l'importance jouée par cette famille protéique au sein de la cellule.

Le gène *SIR2* de *Leishmania major* (*LmSIR2*) codant pour une protéine présentant une forte homologie de séquence avec SIR2 de levure a été caractérisé pour la première fois par le Dr. Ali Ouaissi et son équipe en 1996 (Yahiaoui *et al.* (1996) *Gene* **169**:115-8). Des homologues de cette protéine ont été mis en évidence par Western blot chez toutes les autres espèces de *Leishmania* étudiées (*L. infantum, L. mexicana, L. braziliensis*) ainsi que chez *Trypanosoma cruzi* (Zemzoumi et al. (1998) Biol. Cell. 90:239-45). Cette protéine présente une localisation cytoplasmique hétérogène selon le stade de développement et selon les espèces. Elle est retrouvée associée à des granules cytoplasmiques chez les formes promastigotes et présente une localisation cytoplasmique diffuse chez l'amastigote. La protéine recombinante est également capable de se fixer à la surface des macrophages J774 et d'être internalisée.

Pour connaître le rôle biologique de cette protéine chez le parasite, la surexpression de la protéine LmSIR2 et de son homologue, la protéine LiSIR2, a été réalisée chez *L. infantum.* Les résultats obtenus démontent que l'expression de ces deux protéines augmente la durée de vie des parasites en phase stationnaire; ce phénomène étant corrélé avec une prévention de l'entrée en apoptose des parasites (Vergnes et al. (2002) Gene 296:139-50).

La présente invention a pour objet de fournir une nouvelle souche de protozoaire de la famille des *Trypanosomatidae*, notamment du genre *Leishmania,* de virulence atténuée par inactivation d'un gène dont le caractère de virulence a été nouvellement mis en évidence, ladite souche étant plus efficace que certaines souches connues à ce jour pour induire une immunité contre les infections à protozoaires.

La présente invention a également pour objet l'utilisation de cette souche pour la préparation de médicaments, notamment de vaccins, destinés à prévenir ou à traiter les maladies à protozoaires.

La présente invention concerne une souche de protozoaire de l'espèce *Leishaminia infantum* dont la virulence est inférieure à celle de la souche sauvage correspondante, caractérisée en ce qu'une seule copie du gène *SIR2* présente dans le génome de ladite souche est inactivée.

La virulence d'une souche de protozoaire peut être mesurée par des méthodes bien connues de l'homme de l'art et notamment tel que cela est décrit dans les Exemples 2 et 3 suivants. En particulier, il est possible de mesurer la charge parasitaire au cours du temps d'un animal modèle, tel qu'une souris, infecté par une souche de protozoaire dont on souhaite évaluer la virulence et de la comparer à celle d'un animal modèle similaire infecté par la souche sauvage correspondante. La souche dont on souhaite évaluer la virulence sera dite de virulence inférieure, ou atténuée, si la charge parasitaire de cette souche diminue plus rapidement au cours du temps que celle de la souche sauvage correspondante.

L'inactivation d'un gène peut être réalisée suivant différentes méthodes bien connues de l'homme de l'art et notamment tel que cela est décrit dans l'Exemple 1 de la présente description. En particulier, il est possible de remplacer, par recombinaison homologue, la séquence chromosomique d'un gène donné par une séquence identique dans laquelle la séquence d'un gène de résistance à une drogue de sélection, telle qu'un antibiotique, a été insérée. Alternativement, il est possible de remplacer tout ou partie de la séquence chromosomique d'un gène donné, par la séquence d'un gène de résistance à une drogue de sélection, telle qu'un antibiotique, par recombinaison homologue aux extrémités.

L'identification d'un gène *SIR2* pour une espèce de la famille des *Trypanosomalidae* pour laquelle il n'a pas encore été identifié peut être réalisée selon des techniques bien connues de l'homme de l'art, à partir des séquences déjà connues de *SIR2* d'espèces différentes, et notamment celles de *Leishmania infantum* (GenBank AF487351, SEQ ID NO: 3), *Leishmania amazonensis* (GenBank AF534109, SEQ ID NO: 5), *Leishmania major* (GenBank L40331, SEQ ID NO : 4) et *Trypanosoma brucei* (GenBank AF102869, SEQ ID NO : 6). Si l'on dispose de la séquence complète ou partielle du génome de la souche pour laquelle on souhaite identifier le gène *SIR2,* il est possible d'utiliser un programme de comparaison de séquence du type BLAST (Altschul et al. (1990) J. Mol. Biol. 215:403-410) qui permet de détecter les séquences présentant une similarité de séquence suffisante avec les séquences déjà connues de *SIR2.* Alternativement, comme cela est décrit dans l'Exemple 1 de la présente description, il est aussi possible de fabriquer des sondes, par exemple par PCR, à partir des séquences déjà connues de *SIR2,* de les marquer à l'aide d'un marqueur fluorescent ou radioactif par exemple, et de procéder à un criblage, par exemple selon la méthode de Southern, d'une banque d'ADN complémentaire ou chromosomique d'une souche pour laquelle on souhaite identifier un gène *SIR2.*

Le génome des protozoaires est diploïde, c'est-à-dire que chaque gène est présent en deux copies respectivement présentes sur chacun des chromosomes homologues.

L'utilisation d'une telle souche est avantageuse dans la mesure où l'inactivation d'une seule copie du gène *SIR2* est suffisante pour induire une diminution de la virulence de la souche pour laquelle ce gène a été inactivé.

Une souche protozoaire appartient au genre Trypanosoma ou au genre Leishmania.

Une souche protozoaire appartient au genre Leishmania et notamment aux espèces choisies parmi le groupe comprenant L. donovani, L. major, L. tropica, L. mexicana, L. infantum, L. amazonensis et L. braziliensis.

La présente invention concerne également une composition immunogène caractérisée en ce qu'elle comprend une souche telle que définie ci-dessus.

La présente invention concerne également une composition pharmaceutique, notamment vaccinale, comprenant à titre de substance active une souche telle que définie ci-dessus en association avec un véhicule pharmaceutiquement acceptable.

Selon un mode de réalisation particulier, ladite composition pharmaceutique, notamment vaccinale, comprend en outre un adjuvant, tel qu'une souche de *Mycobacterium vaccae* ou du BCG, de l'alun, du lipide A monophosphorylé, ou de l'interleukine 12.

L'utilisation d'adjuvant est bien connue de l'homme de l'art. Les composés adjuvants permettent notamment d'augmenter la réponse immunitaire de l'organisme auquel est administré une composition immunogène, pharmaceutique ou vaccinale à l'encontre du composé immunogène compris dans ladite composition.

*Mycobacterium vaccae* ou le BCG, sont des agents bactériens vivants, bien connus de l'homme de l'art, susceptibles d'induire une activation du système immunitaire et d'améliorer la réponse immunitaire à l'encontre des protozoaires de l'invention.

Les autres adjuvants susmentionnés sont notamment décrits dans «*Report on the fourth TDR/IDRI Meeting on Second-Generation Vaccines against Leishmaniasis*» de Dumonteil *et al.*, disponible auprès de l'OMS et dans Hadman (2002) Clin. Microbiol. Rev. 14:229-43.

Selon un autre mode de réalisation particulier, ladite composition pharmaceutique, notamment vaccinale convient pour l'administration d'une dose unitaire d'environ $5.10^4$ à environ $5.10^7$ protozoaires par kg.

L'expression « par kg » se réfère à la masse corporelle du sujet à traiter.

Ladite composition pharmaceutique, notamment vaccinale comprend à titre de substance active une souche de Leishmania infantum pour laquelle une copie du gène SIR2 a été inactivée, telle que définie ci-dessus.

Selon un mode de réalisation préféré, ladite composition pharmaceutique, notamment vaccinale, comprend également un ou plusieurs composés utilisables pour la prévention et/ou le traitement des maladies liées à une infection par un protozoaire, telles que les maladies à trypanosomes et les leishmanioses, tels que :

- la miltefosine, les antimoniés, l'amphotéricine B, le benznidazol, le nifurtimox, le pentamidine et ses dérivés, les dérivés arsenicaux, le mélarsopol et le difluorométhylornithine,
- des protozoaires, notamment du genre *Trypanosoma* ou *Leishmania*, inactivés ou de virulence atténuée par inactivation d'un gène différent de *SIR2,*
- des antigènes de protozoaires, tels que les protéines gp46, gp63, LACK, TSA, STI1, Hsp80, SLA, FPA, 1 G6, 4H6, GBP, CPA, CPB, LeIF, A2 ou le lipophosphoglycane, ou
- des séquences d'acides nucléiques codant pour lesdits antigènes protéiques de protozoaires.

Les maladies à trypanosomes correspondent notamment à la maladie du sommeil (*T. brucei*) et à la maladie de Chagas (*T. cruzi).*

Les leishmanioses peuvent être notamment provoquées par *L. donovani*, *L. major*, *L. tropica*, *L. mexicana*, *L. infantum*, *L. amazonensis* ou *L. braziliensis*, et se manifestent sous quatre formes principales : la leishmaniose viscérale (*kala azar*), la leishmaniose cutanée, la leishmaniose muco-cutanée, et la leishmaniose cutanée diffuse.

Avantageusement l'ajout d'un ou plusieurs composés susmentionnés permet d'activer le système immunitaire de l'organisme auquel a été administrée ladite composition de façon synergique avec les souches de protozoaires de l'invention.

Les protozoaires dits inactivés correspondent à des protozoaires tués notamment par autoclavage.

Les protozoaires dits de virulence atténuée par inactivation d'un gène différent de *SIR2*, correspondent en particuliers à des protozoaires, en particulier des *Leishmania*, pour lesquels un des gènes *DHFR-TS*, *CPA*, *CPB*, *A2*, ou *TR* a été inactivé.

Les antigènes de protozoaires susmentionnés correspondent en particulier à des antigènes de *Leishmania* et sont notamment décrit dans «*Report on the fourth TDRI/DRI Meeting on Second-Generation Vaccines against Leishmaniasis*» de Dumonteil *et al.*, disponible auprès de l'OMS, et dans Handman (2002) Clin. Microbiol. Rev. 14:229-43.

L'invention concerne également un produit comprenant :

- au moins une souche telle que définie ci-dessus,
- et au moins un composé utilisable pour la prévention ou le traitement des maladies liées à une infection par un protozoaire, telles que les maladies à trypanosomes et les leishmanioses, tel que :
  - la miltefosine, les antimoniés, l'amphotéricine B, le benznidazol, le nifurtimox, le pentamidine et ses dérivés, les dérivés arsenicaux, le mélarsopol ou le difluorométhylornithine,
  - des protozoaires, notamment du genre *Trypanosoma* ou *Leishmania*, inactivés ou de virulence atténuée par inactivation d'un gène différent de *SIR2,*
  - des antigènes de protozoaires, tels que les protéines gp46, gp63 LACK, TSA, STI1, Hsp80, SLA, FPA, 1G6, 4H6, GBP, CPA, CPB, LeIF, A2 ou le lipophosphoglycane, ou

- des séquences d'acides nucléiques codant pour lesdits antigènes protéiques de protozoaires ;

comme produit de combinaison pour une utilisation simultanée, séparée ou étalée dans le temps pour la prévention et/ou le traitement des maladies liées à une infection par un protozoaire, telles que les maladies à trypanosomes et les leishmanioses.

L'invention concerne également l'utilisation d'une souche telle que définie ci-dessus, pour la préparation d'un médicament, notamment un vaccin, destiné à la prévention et/ou au traitement des leishmanioses.

L'invention concerne également l'utilisation susmentionnée d'une souche de *Leishmania infantum* pour laquelle une copie du gène *SIR2* a été inactivé, telle que définie ci-dessus, pour la préparation d'un médicament, notamment un vaccin, destiné à la prévention et/ou au traitement des leishmanioses, notamment des leishmanioses à *Leishmania infantum.*

Par ailleurs, un mode de réalisation particulier de l'invention prévoit également l'utilisation d'une souche telle que définie ci-dessus, laquelle souche étant de plus tuée, notamment par traitement thermique ou chimique, à l'aide de glutaraldéhyde par exemple, pour la préparation de médicaments ou de vaccins, en particulier dans le cadre de la prévention et/ou du traitement des maladies liées à une infection par un protozoaire, telles que les maladies à trypanosomes et les leishmanioses.

L'invention concerne également les compositions pharmaceutiques comprenant de telles souches tuées, en association avec un véhicule pharmaceutiquement acceptable.

## DESCRIPTION DES FIGURES

**Figures 1A, Figure 1B**

Les figures 1A et 1B représentent les loci des allèles sauvages (*LiSIR2*) (Figure 1A) ou inactivés par les gènes de résistance à la néomycine (*LiSIR2::NEO*) (Figure 1B). Les sites de restriction sont représentés par H : *Hind* III, P : *Pst* I, C : *Cla* I, S : *Sac* I. Les flèches indiquent la taille des fragments de restriction obtenus.

**Figures 2A, Figure 2B**

La figure 2A représente une analyse selon la méthode de transfert de Southern de l'ADN génomique issu de clones sauvages de *L. infantum* digéré par *Pst* I (piste 1), de clones pour lesquels une copie du gène *LiSIR2* a été inactivée (pistes 2 et 3) et hybridé avec la sonde *LiSIR2*.

La figure 2B représente une analyse par Southern Blot de l'ADN génomique de clones sauvages de *L. infantum* digéré par *Sac* I (piste 1), de clones pour lesquels une copie du gène *LiSIR2* a été inactivée (piste 2 et 3), et hybridé avec la sonde *NEO.*

**Figure 3A et Figure 3B**

Les figures 3A et 3B représentent les cinétiques de croissance des parasites sous formes promastigotes (figure 3A) et amastigotes (figure 3B) des différents clones : sauvage (carrés noirs) et *LiSIR2/LiSIR2::NEO* (cercle et triangle blancs) inoculés à $2.10^6$ parasites/ml. L'axe des abscisses représente le temps (en jours) et l'axe des ordonnées le nombre de parasites par ml (à multiplier par log $10^4$ pour la figure 3A et par $10^4$ pour la figure 3B).

**Figure 4A et Figure 4B**

Les figures 4A et 4B représentent l'effet de l'anticorps monoclonal IIIG4 dirigé contre la protéine SIR2 de *L. major* sur la croissance des amastigotes de *L. infantum* sauvages (figure 4A) ou pour lesquels une copie du gène *LiSIR2* a été inactivée (figure 4B). Dans la figure 4A la courbe figurée par les triangles noirs représente l'effet de IIIG4 dilué au 1/20 et la courbe figurée par les carrés noirs l'effet de l'anticorps témoin IIIB9F10 dirigé contre une protéine flagellaire de *T. cruzi* (la Tc24). Dans la figure 4B, les courbes figurées respectivement par les cercles blancs, les carrés blancs, les cercles noirs et les carrés noirs représentent l'effet de IIIG4 dilué au 1/20, de IIIB9F10, de IIIG4 dilué au 1/40 et un témoin.

**Figures 5A et 5B**

La figure 5A représente la variation par rapport aux parasites sauvages (en pourcentage, axe des ordonnées) de la quantité de parasites en phase G0/G1 (1), en phase S (2) ou en phase G2/M (3) pour des parasites sur-exprimant la protéine SIR2 de *L. major* (colonne blanche), ou ayant une copie du gène *SIR2* inactivée (*LiSIR2/LiSIR2::NEO*) (colonne noire).

La figure 5B représente le pourcentage de parasites en apoptose pour des parasites sauvages (1), ayant une copie du gène *SIR2* inactivée (*LiSIR2*/*LiSIR2::NEO*) (2) ou sur-exprimant la protéine SIR2 de *L. major* (3), en présence de Sirtinol à 25 μg/ml (un inhibiteur de SIR2) et de YOPRO-1 (un marqueur d'apoptose, Idzioreck et al. (1995) J. Immunol. Methods 185:249-258).

**Figures 6A et 6B**

La figure 6A représente la réduction de l'incorporation d'uracile tritiée (axe des ordonnées, en pourcent) chez des macrophages dérivés de la lignée TPH1 infectés par des souches mutantes indépendantes du type *LiSIR2*/*LiSIR2::NEO* (colonnes blanches et noires) par rapport à des macrophages infectés par une souche sauvage.

La figure 6B représente la charge parasitaire dans la rate de souris BALB/c infectées (axe des ordonnées, log du nombre de parasites par gramme) soit par une souche de *L. infantum* sauvage (losanges noirs) soit par une souche de *L. infantum* pour laquelle une copie du gène *SIR2* a été inactivée (*LiSIR2+*/- carrés blancs), en fonction du temps (axe des abscisses, en semaines), on observe une réduction progressive de la charge parasitaire chez les souris infectées par les clones mutants et aucun parasite vivant n'a pu être détecté 6 semaines après infection.

**Figure 7**

La figure 7 représente la charge parasitaire dans la rate de souris BALB/c infectées (axe des ordonnées, log du nombre de parasites par gramme) soit par une souche de *L. infantum* sauvage (barre noire) soit par une souche de *L. infantum* pour laquelle une copie du gène *SIR2* a été inactivée (*LiSIR2*+/- barre blanche), puis réinfectées après 6 semaines par une souche de *L. infantum* sauvage. La charge parasitaire est déterminée 6 semaines après la réinfection. On observe une réduction significative de la charge parasitaire chez les souris ayant été primo-infectées par les clones mutants.

## EXEMPLES

La présente invention découle de la mise en évidence par les inventeurs du caractère virulent du gène *SIR1* chez les protozoaires de la famille des *Trypanosomatidae.*

## EXEMPLE 1

### Obtention d'une souche de *Leishmania infantum* pour laquelle le gène *SIR2* est inactivé

#### 1. Inactivation du gène *SIR2* de *L. infantum* (*LiSIR2*)

Le gène unique *LiSIR2* a été inactivé chez le parasite par recombinaison homologue.

Les souches promastigotes de *L. infantum* (clone MHOM/67/ITMAP-263) et les clones mutants qui en dérivent ont été obtenus par dilution limite et mis en culture à 26°C dans un milieu SDM-79 (Brun et al. (1979) Acta Trop. 36: 289-292) supplémenté avec 10% de sérum de veau foetal inactivé à 56°C, 100 U/ml de pénicilline et 100 μg/ml streptomycine et 10 μg/ml de néomycine (G418) pendant la période de sélection des clones recombinants. Les populations clonales d'amastigotes axéniques ont été maintenues à 36 +/- 1 °C en présence de 5% $CO_2$ dans un milieu acellulaire MAA/20 (Sereno et al. (1997) Antimicrob. Agents Chemother. 41:972-976).

Une banque d'ADN génomique de *L. infantum* a été criblée avec la sonde portant la séquence du gène *SIR2* de *L. major* afin d'isoler un grand fragment génomique contenant le gène *LiSIR2* et ses régions flanquantes.

Brièvement, le gène *SIR2* de *L. major* (Yahiaoui et al. (1996) Gene 169:115-8) a été amplifié par PCR en utilisant deux amorces spécifiques *LmSIR2-A* (5'-gaattcGATATGACAGGGTCTCCG-3') (SEQ ID NO: 1) et *LmSIR2-B* (5'-ctcgagCAGTCACCATGTTGGCAG-3') (SEQ ID NO : 2). Le fragment amplifié de 1200 pb obtenu a été cloné dans le vecteur pCR2.1 en utilisant le kit de clonage commercial « TA cloning kit » (Invitrogen). Le fragment radiomarqué avec du α($^{32}$P)dCTP a été utilisé pour cribler la banque génomique de *L. infantum* en utilisant des protocoles standards (Yahiaoui et al. (1996) Gene 169:115-8) bien connus de l'homme de l'art. Des clones positifs ont été analysés par des enzymes de restriction et analysés selon la méthode de transfert de Southern en utilisant la même sonde radiomarquée. Un fragment de 6kb généré par l'enzyme *Hind* III portant le gène *LiSIR2* a été sous cloné dans le plasmide pUC18 (désignation : pUC-SIR) et séquencé. La détermination des cadres de lecture a permis d'identifier une séquence nucléotidique de 1119 pb (SEQ ID NO : 3) codant pour une protéine de 373 aa montrant 93% d'identité avec le gène *LmSIR2* de *L. major.* Cette séquence a été soumise à GenBank sous le code AF487351. Une représentation schématique de l'allèle sauvage portant le fragment de 6 kb est donnée dans la **Figure 1A**. Le plasmide pUC-SIR a été ouvert par l'enzyme *Cla* I ayant un site unique dans le gène *LiSIR2* et traité successivement par la nucléase de haricot mungo et la phosphatase afin de générer des bouts francs déphosphorylés. La cassette contenant le gène codant pour la néomycine phosphotransferase (*NEO*) utilisée pour la construction du vecteur pour l'inactivation a été dérivée de la construction pSP*Yneo* (Papadopoulou et al. (1994) J. Biol. Chem. 269:7310-7315). La cassette *NEO* sous le contrôle des régions riches en bases pyrimidiques a été isolée par une digestion par *Bam* HI-*Bgl* II et traitées par la polymérase de Klenow pour générer des bouts francs. Le gène de résistance à la néomycine a alors été intégrés dans le site unique *Cla* I présent dans la séquence codante de *LiSIR2* (**Figures 1B**). La construction linéarisée par *Hind* III a ensuite été électroporée chez les promastigotes de la phase de croissance logarithmique du clone sauvage *L. infantum* MON1 (clone : MHOM/67/ITMAP-263). Après sélection, les parasites transformants ont été clonés par dilution limite et caractérisés.

Brièvement, les promastigotes ont été transfectés par électroporation avec 1μg d'ADN linéarisé purifié sur gel d'agarose comme décrit précédemment (450 V.$cm^{-1}$ 450 μF) (Vergnes et al. (2002) Gene 296:139-50). Dans un premier temps les promastigotes électroporés ont été incubés dans 5 ml de milieu SDM-79 sans ajout d'antibiotique de sélection pendant 24 h. Après cette étape, l'antibiotique néomycine (G418) a été rajouté dans le milieu de culture à raison de 10 μg/ml pendant deux semaines. Les parasites obtenus ont été clonés par la méthode de dilution limite en présence de 10 μg/ml de G418.

**2. Caractérisation moléculaire des mutants *LiSIR2+/-***

L'inactivation d'un allèle du gène *LiSIR2* a été réalisée en intégrant la construction contenant le gène de résistance à la néomycine (mutant *LiSIR2*+/- ou *LiSIR2*/*LiSIR2::NEO*). Le Southern blot révèle chez tous les clones recombinants la présence d'un allèle sauvage à 4kb et deux fragments supplémentaires à 2.8kb et 2.5kb correspondant à l'allèle *LiSIR2::NEO,* en accord avec le profil de restriction obtenu par *Pst* I (**Figures 1A, 1B et 2A**).

**3. Caractérisation phénotypique des parasites mutants *LiSIR2+/-***

*a. LiSIR2* est indispensable à la multiplication des formes amastigotes axéniques

Pour étudier l'effet de l'inactivation d'un allèle du gène *LiSIR2* sur la prolifération des parasites en culture axénique, les Inventeurs ont réalisé des cinétiques de croissance sur les deux stades parasitaires en utilisant l'iodure de propidium comme marqueur de viabilité. Les courbes de croissance du clone sauvage et des mutants ont été réalisées en inoculant le milieu de culture SDM-79 ou MAA/20 avec respectivement $2.10^6$ ou $4.10^6$ promastigotes ou amastigotes ; les comptages ont été réalisés à des intervalles de 24 h comme décrit précédemment (Vergnes et al. (2002) Gene 296: 139-50). L'inactivation d'un allèle du gène *LiSIR2* n'affecte pas la croissance des parasites sous forme promastigote (**Figure 3A**). On observe par contre chez ces mêmes parasites sous forme amastigote, une forte diminution de leur capacité à proliférer en culture par rapport aux parasites sauvages (**Figure 3B**).

Pour confirmer l'implication directe du niveau de synthèse de LiSIR2 dans la capacité de prolifération des parasites sous forme amastigote, des cinétiques de croissance d'amastigotes sauvages (**Figure 4A**) ou ayant perdu une copie du gène *LiSIR2* (**Figure 4B**) ont été réalisées en présence de l'anticorps monoclonal IIIG4 (Vergnes et al. (2002) Gene 296:139-50) dirigé contre la protéine LmSIR2. Un anticorps monoclonal IIIB9F10 (Ouaissi et al. (1992) Biol. Cell. 75:11-17) de même isotype dirigé contre la protéine Tc24 de *Trypanosoma cruzi* a été utilisé comme contrôle. L'ajout de l'anticorps IIIG4 à la dilution 1/20 ralentit considérablement la croissance des parasites sauvages, avec l'apparition d'une phase de latence très prolongée. Les mêmes parasites en présence du IIIB9F10 à une concentration identique ont une cinétique de croissance comparable à celle du clone sauvage (**Figure 4A**). La même expérience réalisée avec un clone parasitaire dépourvu d'une copie du gène *LiSIR2* (clone *LiSIR2* +/-) montre que l'anticorps IIIG4 utilisé au 1/20 bloque totalement la prolifération et tue les parasites. Le même anticorps dilué au 1/40 ou le IIIB9F10 dilué au 1/20 ne modifient pas la cinétique de croissance des amastigotes par rapport au clone contrôle *LiSIR2 +/-.* L'action du IIIG4 semble donc être dose-dépendante ce qui suggère que la protéine LiSIR2 est indispensable aux amastigotes pour se multiplier.

b. L'inactivation d'un allèle du gène *LiSIR2* bloque les parasites en phase G0/G1 du cycle cellulaire

Pour connaître le mode d'action de LiSIR2 chez les formes amastigotes, des études de cycle cellulaire ont été réalisées sur les parasites mutants ayant différents niveaux d'expression de la protéine LiSIR2 par rapport aux parasites sauvages. Les clones inactivés *LiSIR2*/*LiSIR2::NEO* possèdent un fort pourcentage de cellules bloquées en phase G0/G1 du cycle (**Figure 5A**). En revanche, on observe un enrichissement des cellules en phase G2/M chez les parasites surexprimant la protéine LmSIR2 (clone pTEX-LmSIR2).

D'autres expériences ont été réalisées en utilisant le Sirtinol (Calbiochem) qui est un inhibiteur spécifique des protéines SIR2. On observe à une concentration de *25* μg/ml un enrichissement des parasites en phase G0/G1 avec l'apparition chez le clone 2 d'un pic pré-G0/G1 qui correspond à la fragmentation de l'ADN génomique observée lors de la phase finale de l'apoptose. Les clones sauvages et surexprimant présentent des phénotypes respectivement intermédiaires. L'utilisation du YOPRO-1, un marqueur d'apoptose (Idzioreck et al. (1995) J. Immunol. Methods 185: 249-258), sur ces mêmes parasites en présence de Sirtinol montre que le niveau d'expression de la protéine LiSIR2 est directement proportionnel au pourcentage d'apoptose observé chez les parasites (**Figure 5B**).

**EXEMPLE 2**

**Virulence atténuée des parasites mutants *in vitro***

La virulence de plusieurs clones ayant perdu un allèle du gène *LiSIR2* a été déterminée *in vitro* en comparant le niveau d'incorporation d'Uracile $^3$H entre des macrophages infectés par des parasites sauvages et infectés par les souches mutants. L'infection des macrophages dérivés de la lignée THP1 par des amastigotes a été réalisée en routine comme décrit précédemment (Sereno et al. (1998) Antimicrob. Agents Chemother. 42:3097-3102). On observe chez tous les clones analysés *LiSIR2/LiSIR2::NEO* une réduction significative d'environ 50% du niveau d'incorporation par rapport aux parasites sauvages (**Figure 6A**).

**EXEMPLE 3**

**Virulence atténuée des parasites mutants *in vivo***

La virulence de ces parasites a également été déterminée *in vivo* en mesurant la charge parasitaire dans la rate de souris BALB/c infectées soit par le clone *L. infantum* sauvage (WT) soit par un clone dépourvu d'une copie du gène *LiSIR2* (*LiSIR2*+/-).

Des souris mâles âgées de 7 semaines (Harlan Iberica, Espagne) ont été infectées par voie intrapéritonéale *(i. p)* avec $10^8$ promastigotes du clone mutant dans lequel une copie du gène *LiSIR2* a été inactivée (*LiSIR2*+/-), comme contrôle, des souris de même âge et sexe ont été infectées par voie intra péritonéale (*i.p.*) avec $10^8$ promastigotes du clone sauvage. Les rates prélevées des deux groupes de souris ont été homogénéisées et soumises à une dilution sériée (1 à $4.10^{-6}$) dans des plaques de microtitration de 96 puits. Après 15 jours d'incubation à 26°C la présence ou l'absence de promastigotes dans les puits de culture a été enregistrée. Le titre est la dernière dilution qui permet d'objectiver un parasite/par puits. Le nombre de parasites par gramme de tissu (charge parasitaire) est calculé comme suit:

**Charge parasitaire = (moyenne géométrique de l'inverse du titre de chaque puits en triplicat / le poids de l'homogénat de rate) x l'inverse de la fraction de l'homogénat de la rate inoculé dans le premier puits de la plaque de culture.**

Bien que les clones *LiSIR2+/-* soient infectieux (charge parasitaire sensiblement identique dans les deux cas après deux semaines d'infection), on observe une réduction progressive de la charge parasitaire chez les souris infectées par les clones mutants et aucun parasite vivant n'a pu être détecté 6 semaines après infection (**Figure 6B**), ce qui fait de ces clones des souches particulièrement adaptées à la préparation d'un vaccin vivant atténué.

**Exemple 4**

**Vaccination de souris par les parasites mutants**

Les clones *LiSIR2*+l- ont été utilisés afin de procéder à des essais de vaccination de souris BALB/c.

Un groupe de souris mâles âgées de 7 semaines (Harlan Iberica, Espagne) (n = 3) a été infecté par voie intrapéritonéale avec $10^8$ promastigotes du clone mutant, dans lequel une copie du gène *LiSIR2* a été inactivée (*LiSIR2*+/-). Comme contrôle, un autre groupe de souris (n = 4) de même âge et sexe a été infecté avec $10^8$ promastigotes du clone sauvage.

Au bout de 6 semaines, les animaux de chaque groupe ont reçu une infection d'épreuve de $10^8$ promastigotes du clone sauvage.

Puis, 6 semaines plus tard, la charge parasitaire a été mesurée comme cela est décrit dans **l'Exemple 3**.

Les résultats obtenus (**Figure 7**) indiquent que la charge parasitaire moyenne est d'environ 20 000 parasites par gramme de tissu pour le groupe de souris ayant reçu une injection du clone *LiSIR2+/-* et d'environ 440 000 000 parasites par gramme de tissu pour l'autre groupe. Ceci confirme le potentiel vaccinant des souches *LiSIR2+/-* de l'invention.

Par ailleurs, l'effet protecteur induit par des parasites *LiSIR2+/-* inactivés, c'est-à-dire tués, par le glutaraldéhyde est également étudié.

Enfin, des expériences de vaccination similaires à celles décrites ci-dessus, impliquant un plus grand nombre de souris (n = 12) sont également réalisées soit avec les clones mutés, soit avec les clones mutés inactivés, afin de mesurer différents paramètres après infection, tel que la charge parasitaire, la production d'anticorps anti-parasite (classe et sous-classe), les niveaux de prolifération cellulaires, ainsi que le profil de sécrétion des cytokines.

**Revendications**

**1.** Souche de protozoaire de l'espèce *Leishmania infantum* dont la virulence est inférieure à celle de la souche sauvage correspondante, **caractérisée en ce qu'**une seule copie du gène *SIR2* présente dans le génome de ladite souche est inactivée.

**2.** Composition immunogène **caractérisée en ce qu'**elle comprend une souche selon la revendication 1.

**3.** Composition pharmaceutique, notamment vaccinale, comprenant à titre de substance active une souche selon la revendication 1 en association avec un véhicule pharmaceutiquement acceptable.

**4.** Composition pharmaceutique, notamment vaccinale, selon la revendication 3, comprenant en outre au moins un adjuvant, tel qu'une souche de *Mycobacterium vaccae* ou du BCG, de l'alun, du lipide A monophosphorylé, ou de l'interleukine 12.

**5.** Composition pharmaceutique, notamment vaccinale, selon la revendication 3 ou 4, convenant pour l'administration d'une dose unitaire d'environ $5.10^4$ à environ $5.10^7$ protozoaires par kg.

**6.** Composition pharmaceutique, notamment vaccinale, selon l'une des revendications 3 à 5, comprenant également un ou plusieurs composés utilisables pour la prévention et/ou le traitement des maladies liées à une infection par un protozoaire, telles que les maladies à trypanosomes et les leishmanioses, tels que :

- la miltefosine, les antimoniés, l'amphotéricine B, le benznidazol, le nifurtimox, le pentamidine et ses dérivés, les dérivés arsenicaux, le mélarsopol et le difluorométhylornithine,
- des protozoaires, notamment du genre *Trypanosoma* ou *Leishmania,* inactivés ou de virulence atténuée par inactivation d'un gène différent de *SIR2,*
- des antigènes de protozoaires, tels que les protéines gp46, gp63, LACK, TSA, STI1, Hsp80, SLA, FPA, 1G6, 4H6, GBP, CPA, CPB, LeIF, A2 ou le lipophosphoglycane, ou
- des séquences d'acides nucléiques codant pour lesdits antigènes protéiques de protozoaires.

**7.** Produit comprenant :

• au moins une souche selon la revendications 1,
• et au moins un composé utilisable pour la prévention ou le traitement des maladies liées à une infection par un protozoaire, telles que les maladies à trypanosomes et les leishmanioses, tel que :

  - la miltefosine, les antimoniés, l'amphotéricine B, le benznidazol, le nifurtimox, le pentamidine et ses dérivés, les dérivés arsenicaux, le mélarsopol et le difluorométhylornithine,
  - des protozoaires, notamment du genre *Trypanosoma* ou *Leishmania,* inactivés ou de virulence atténuée par inactivation d'un gène différent de SIR2,
  - des antigènes de protozoaires, tels que les protéines gp46, gp63, LACK, TSA, STI1, Hsp80, SLA, FPA, 1G6, 4H6, GBP, CPA, CPB, LeIF, A2 ou le lipophosphoglycane, ou
  - des séquences d'acides nucléiques codant pour lesdits antigènes protéiques de protozoaires ;

comme produit de combinaison pour une utilisation simultanée, séparée ou étalée dans le temps pour la prévention et/ou le traitement des maladies liées à une infection par un protozoaire, telles que les maladies à trypanosomes et les leishmanioses.

**8.** Utilisation d'une souche selon la revendication 1 pour la préparation d'un médicament, notamment un vaccin, destiné à la prévention et/ou au traitement des leishmanioses.

## Claims

**1.** Protozoan strain of the species *Leishmania infantum* the virulence of which is lower than that of the corresponding wild-type strain, **characterized in that** only one copy of the *SIR2* gene present in the genome of said strain is inactivated.

**2.** Immunogenic composition **characterized in that** it comprises a strain according to claim 1.

**3.** Pharmaceutical, in particular vaccinal, composition, comprising as active ingredient a strain according to claim 1, in combination with a pharmaceutically acceptable vehicle.

**4.** Pharmaceutical, in particular vaccinal, composition, according to claim 3, also comprising at least one adjuvant, such as a strain of *Mycobacterium vaccae* or BCG, alum, monophosphorylated lipid A, or interleukin 12.

**5.** Pharmaceutical, in particular vaccinal, composition, according to claim 3 or 4, suitable for the administration of a single dose of approximately $5.10^4$ to approximately $5.10^7$ protozoans per kg.

**6.** Pharmaceutical, in particular vaccinal, composition, according to any one of claims 3 to 5 , also comprising one or more compounds which can be used for the prevention and/or treatment of diseases linked to a protozoan infection, such as diseases caused by trypanosomes and leishmaniae, such as:

- miltefosine, antimonials, amphotericin B, benznidazole, nifurtimox, pentamidine and its derivatives, arsenic derivatives, melarsopol and difluoromethylornithine,
- protozoans, in particular of the genus *Trypanosoma* or *Leishmania,* inactivated or of reduced virulence by inactivation of a gene different from SIR2,
- protozoan antigens, such as the proteins gp46, gp63, LACK, TSA, STI1, Hsp80, SLA, FPA, 1G6, 4H6, GBP, CPA, CPB, LeIF, A2 or lipophosphoglycan, or
- nucleic acid sequences encoding said protozoan antigen proteins.

**7.** Product comprising:

• at least one strain according to claim 1,
• and at least one compound which can be used for the prevention or the treatment of the diseases linked to a protozoan infection, such as the diseases caused by trypanosomes and leishmaniae, such as:

  - miltefosine, antimonials, amphotericin B, benznidazole, nifurtimox, pentamidine and its derivatives, arsenic derivatives, melarsopol or difluoromethylornithine,
  - protozoans, in particular of the genus *Trypanosoma* or *Leishmania,* inactivated or of reduced virulence by inactivation of a gene different from SIR2,
  - protozoan antigens, such as the proteins gp46, gp63, LACK, TSA, STI1, Hsp80, SLA, FPA, 1G6, 4H6, GBP, CPA, CPB, LeIF, A2 or lipophosphoglycan, or
  - sequences of nucleic acids encoding said protozoan antigen proteins;

as a combination product for simultaneous or separate use or use spread out over time for the prevention and/or the treatment of the diseases linked to a protozoan infection, such as the diseases caused by trypanosomes and leishmaniae.

**8.** Use of a strain according to claim 1 for the preparation of a drug, in particular a vaccine, for the prevention and/or the treatment of leishmaniae.

## Patentansprüche

1. Protozoenstamm der Spezies *Leishmania infantum,* dessen Virulenz geringer ist, als die des entsprechenden Wildtyp-Stamms, **dadurch gekennzeichnet, dass** eine einzige Kopie des Gens *SIR2,* das in dem Genom des Stamms vorhanden ist, inaktiviert ist.

2. Immunogene Zusammensetzung, **dadurch gekennzeichnet, dass** sie einen Stamm nach Anspruch 1 umfasst.

3. Pharmazeutische Zusammensetzung, insbesondere Impfzusammensetzung, die als Wirkstoff einen Stamm nach Anspruch 1 in Assoziation mit einem pharmazeutisch verträglichen Vehikel umfasst.

4. Pharmazeutische Zusammensetzung, insbesondere Impfzusammensetzung, nach Anspruch 3, die ferner mindestens ein Adjuvans, wie etwa einen *Mycobacterium*vaccae-Stamm oder BCG, Alaun, monophosphoryliertes Lipid A oder Interleukin 12 umfasst.

5. Pharmazeutische Zusammensetzung, insbesondere Impfzusammensetzung, nach Anspruch 3 oder 4, die für die Verabreichung einer Einzeldosis von etwa $5.10^4$ bis etwa $5.10^7$ Protozoen pro kg geeignet ist.

6. Pharmazeutische Zusammensetzung, insbesondere Impfzusammensetzung, nach einem der Ansprüche 3 bis 5, die außerdem eine oder mehrere Verbindungen umfasst, die für die Vorbeugung und/oder die Behandlung von Krankheiten verwendbar sind, die mit einer Infektion mit einem Protozoen verbunden sind, wie etwa durch Trypanosomen verursachte Erkrankungen und Leishmaniosen, wie etwa:

   - Miltefosin, Antimon enthaltende Präparate, Amphotericin B, Benznidazol, Nifurtimox, Pentamidin und dessen Derivate, Arsenderivate, Melarsopol und Difluormethylornithin,
   - Protozoen, insbesondere der Gattung *Trypanosoma* oder *Leishmania,* die inaktiviert sind oder durch Inaktivierung eines Gens, das von *SIR2* verschieden ist, eine abgeschwächte Virulenz aufweisen,
   - Protozoenantigene, wie etwa die Proteine gp46, gp63, LACK, TSA, STI1, Hsp80, SLA, FPA, 1G6, 4H6, GBP, CPA, CPB, LeIF, A2 oder Lipophosphoglycan, oder
   - Nukleinsäuresequenzen, die für die Protozoenantigenproteine kodieren.

7. Produkt, umfassend:

   • mindestens einen Stamm nach Anspruch 1,
   • und mindestens eine Verbindung, die zur Vorbeugung oder Behandlung von Erkrankungen verwendbar ist, die mit einer Infektion durch einen Protozoen verbunden sind, wie etwa durch Trypanosomen verursachte Erkrankungen und Leishmaniosen, wie etwa:

     - Miltefosin, Antimon enthaltende Präparate, Amphotericin B, Benznidazol, Nifurtimox, Pentamidin und dessen Derivate, Arsenderivate, Melarsopol und Difluormethylornithin,
     - Protozoen, insbesondere der Gattung *Trypanosoma* oder *Leishmania,* die inaktiviert sind oder durch Inaktivierung eines Gens, das von *SIR2* verschieden ist, eine abgeschwächte Virulenz aufweisen,
     - Protozoenantigene, wie etwa die Proteine gp46, gp63, LACK, TSA, STI1, Hsp80, SLA, FPA, 1G6, 4H6, GBP, CPA, CPB, LeIF, A2 oder Lipophosphoglycan, oder
     - Nukleinsäuresequenzen, die für die Protozoenantigenproteine kodieren;

   als Kombinationsprodukt für eine Verwendung, die gleichzeitig, getrennt oder mit verzögerter Freisetzung erfolgt, zur Vorbeugung und/oder Behandlung von Krankheiten, die mit einer Infektion durch einen Protozoen verbunden sind, wie etwa von Trypanosomen verursachte Erkrankungen und Leishmaniosen.

8. Verwendung eines Stamms nach Anspruch 1 zur Herstellung eines Medikaments, insbesondere eines Impfstoffs, der zur Vorbeugung und/oder Behandlung von Leishmaniosen bestimmt ist.

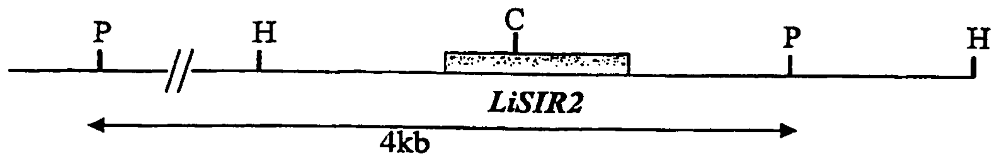


FIGURE 1A

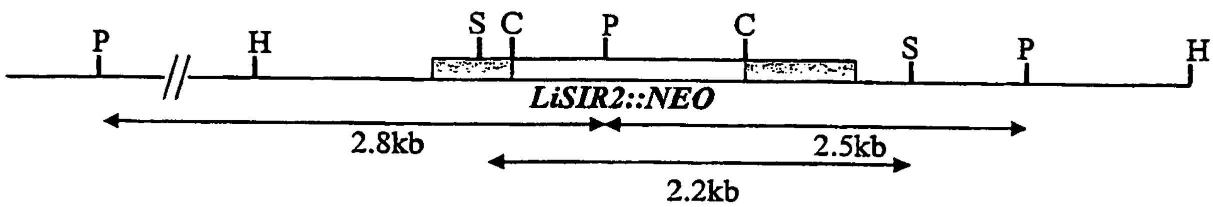


FIGURE 1B

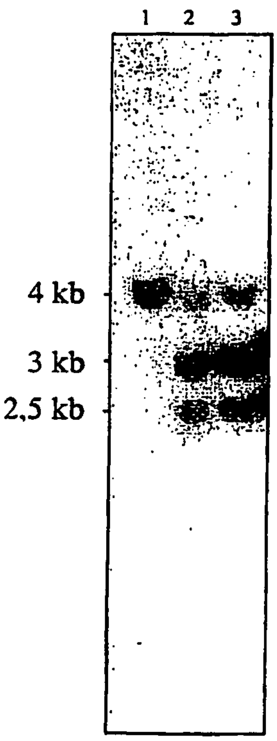


FIGURE 2A

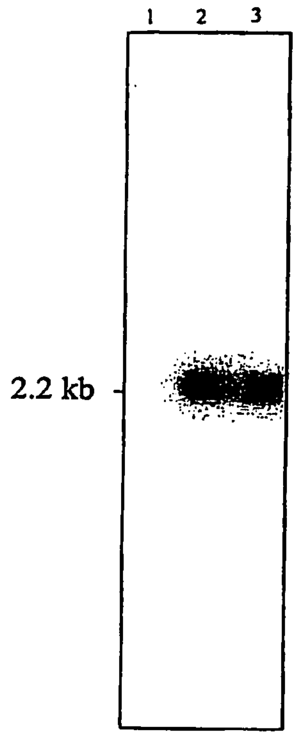


FIGURE 2B

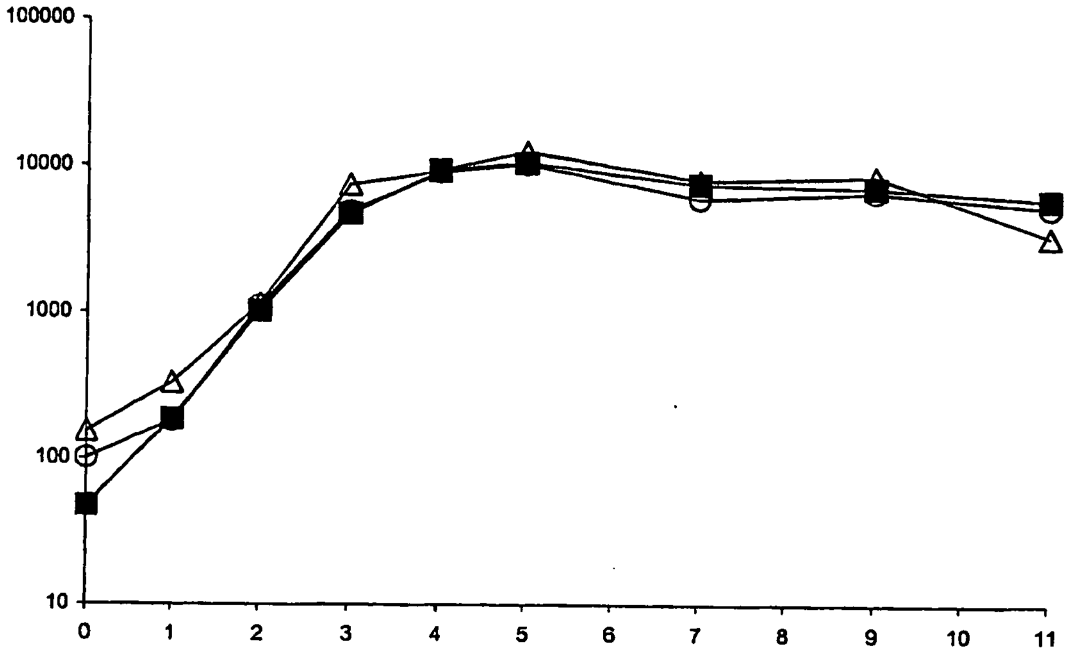


FIGURE 3A

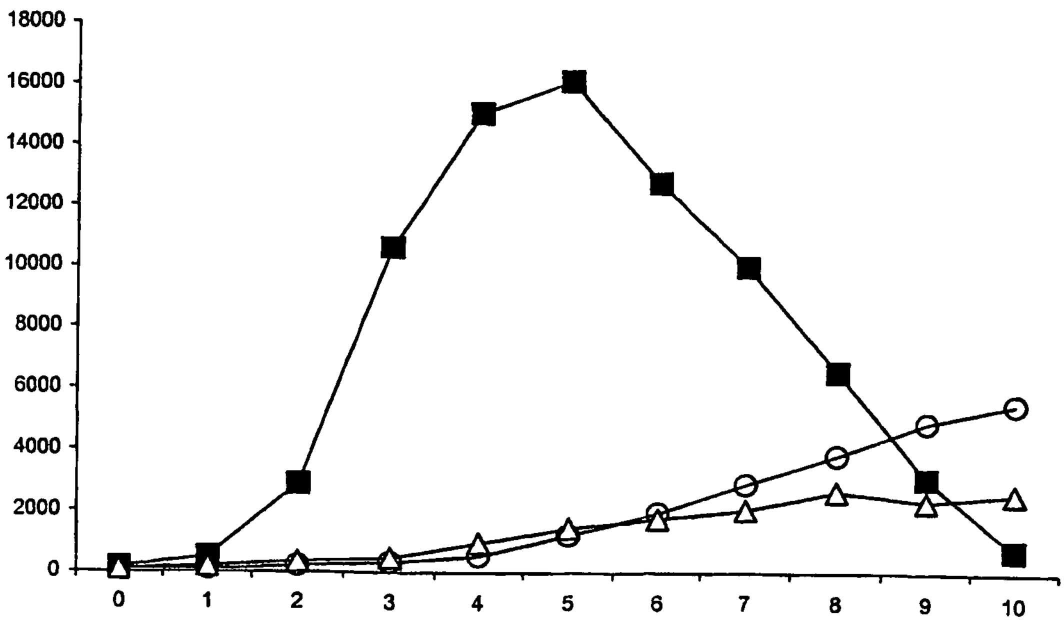


FIGURE 3B

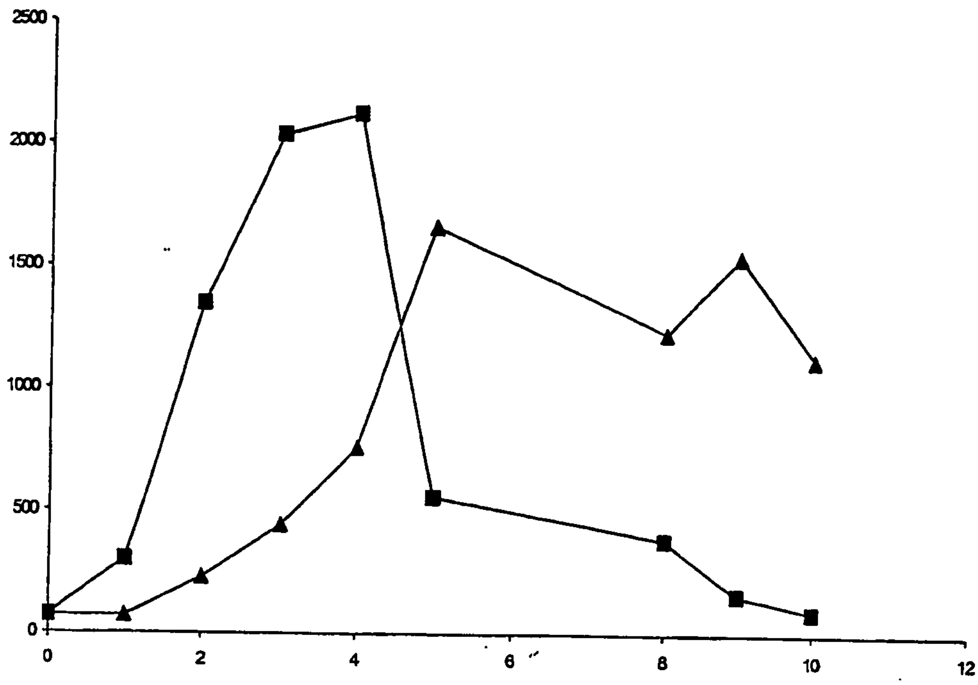


FIGURE 4A

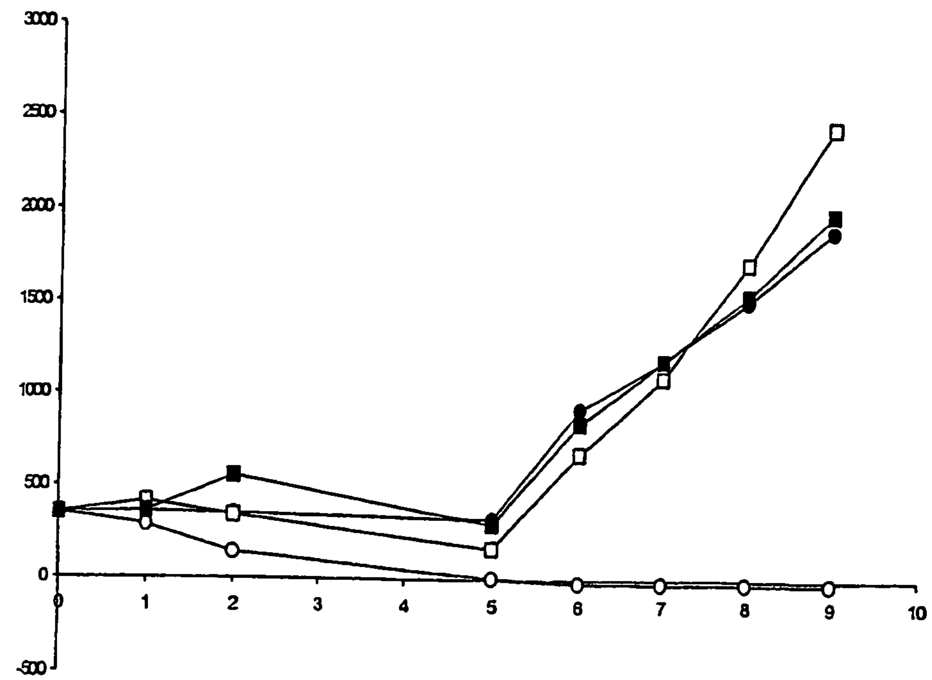


FIGURE 4B

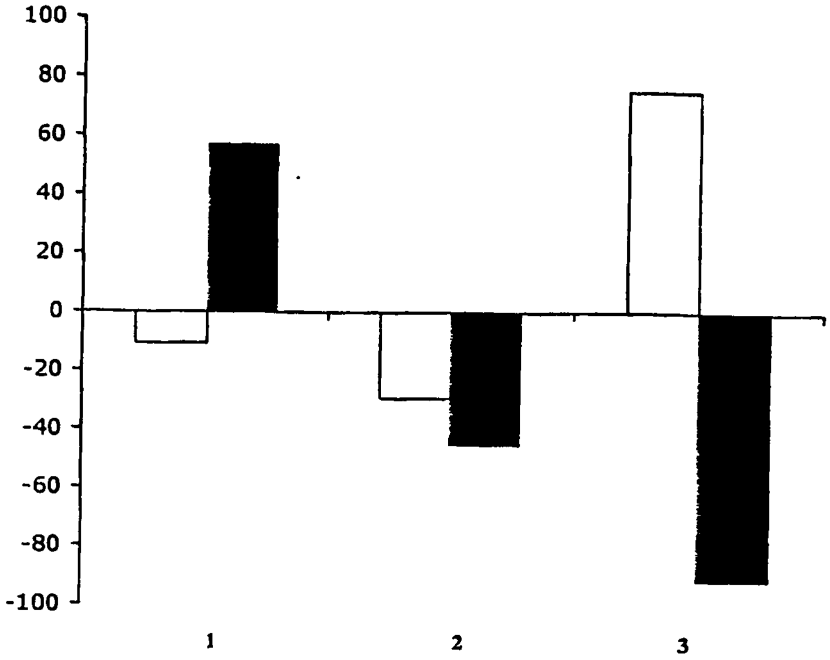


FIGURE 5A

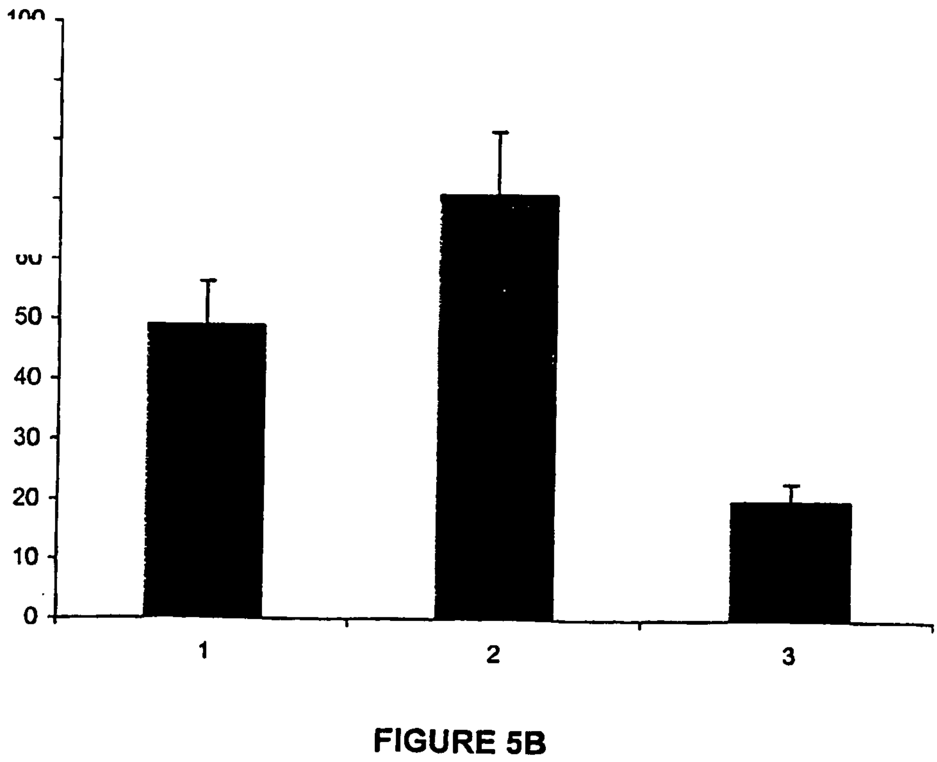


FIGURE 5B

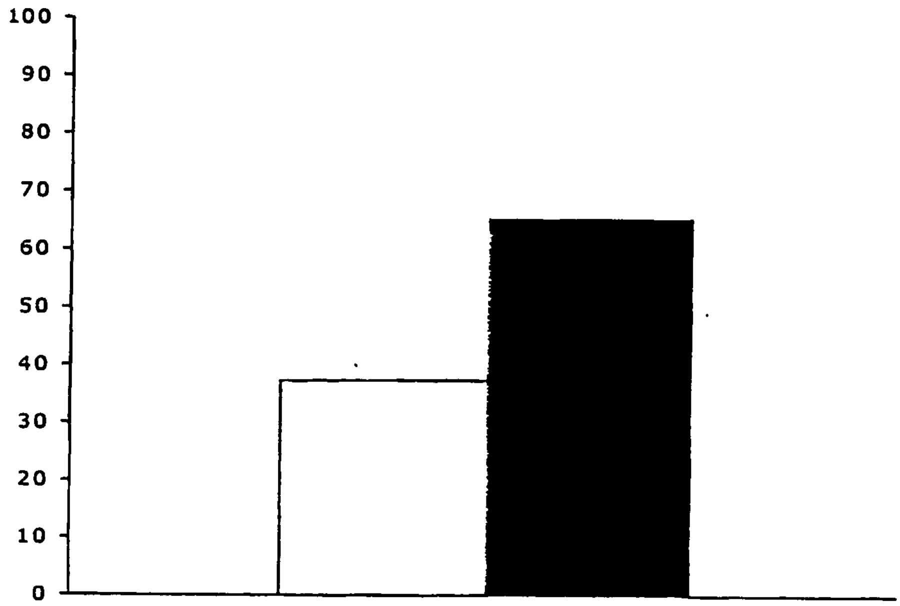


FIGURE 6A

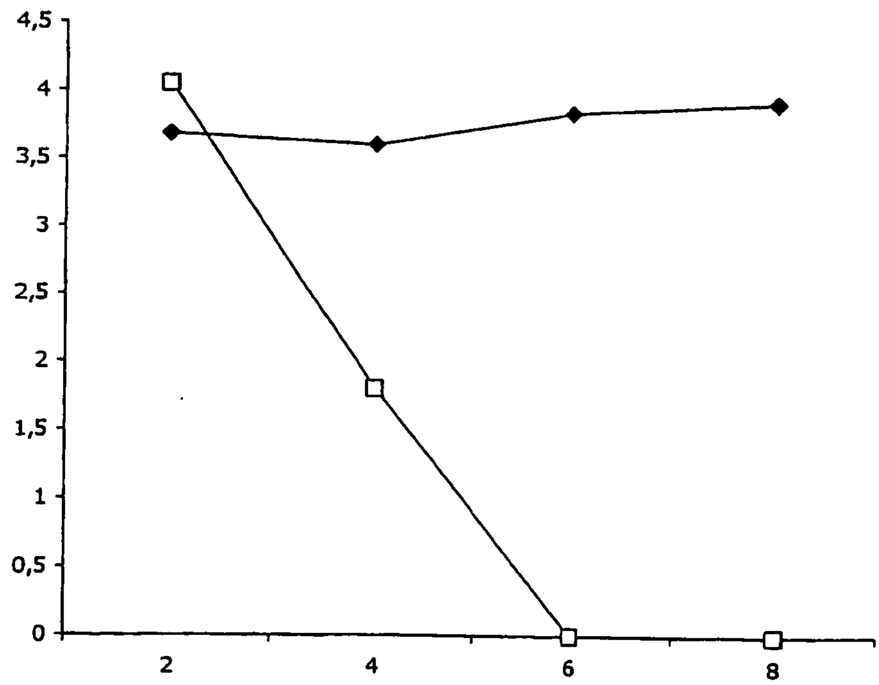


FIGURE 6B

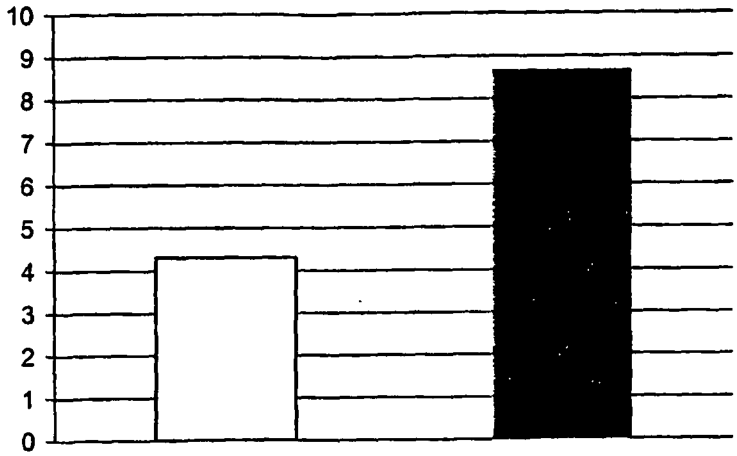


Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9844943 A
- EP 0716697 A
- EP 0806476 A


### Littérature non-brevet citée dans la description


- **TITUS et al.** *Proc. Natl. Acad Sci. USA,* 1995, vol. 92, 10267-10271
- **GUARENTE et al.** *Nat. Genet.,* 1999, vol. 23, 281-5
- **GARTENBERG et al.** *Curr. Opin. Microbiol.,* 2000, vol. 3, 132-7
- **MIN et al.** *Cell,* 2001, vol. 105, 269-79
- **TSUKAMOTO et al.** *Nature,* 1997, vol. 388, 900-3
- **GUARENTE et al.** *Genes Dev.,* 2000, vol. 14, 1021-6
- **TISSENBAUM et al.** *Nature,* 2001, vol. 410, 227-30
- **FRYE et al.** *Biochem. Biophys. Res. Commun.,* 2000, vol. 273, 793-8
- **VAZIRI et al.** *Cell,* 2001, vol. 107, 149-59
- **LUO et al.** *Cell,* 2001, vol. 107, 137-48
- **MUTH et al.** *Embo J.,* 2001, vol. 20, 1353-62
- **BELL et al.** *Science,* 2002, vol. 296, 148-51
- **BERESHCHENKO et al.** *Nat. Genet.,* 2002, vol. 32, 606-13
- **ZEMZOUMI et al.** *Biol. Cell,* 1998, vol. 90, 239-45
- **VERGNES et al.** *Gene,* 2002, vol. 296, 139-50
- **ALTSCHUL et al.** *J. Mol. Biol.,* 1990, vol. 215, 403-410
- **HADMAN.** *Clin. Microbiol. Rev.,* 2002, vol. 14, 229-43
- **HANDMAN.** *Clin. Microbiol. Rev.,* 2002, vol. 14, 229-43
- **IDZIORECK et al.** *J. Immunol. Methods,* 1995, vol. 185, 249-258
- **BRUN et al.** *Acta Trop.,* 1979, vol. 36, 289-292
- **SERENO et al.** *Antimicrob. Agents Chemother.,* 1997, vol. 41, 972-976
- **YAHIAOUI et al.** *Gene,* 1996, vol. 169, 115-8
- **PAPADOPOULOU et al.** *J. Biol. Chem.,* 1994, vol. 269, 7310-7315
- **OUAISSI et al.** *Biol. Cell,* 1992, vol. 75, 11-17
- **SERENO et al.** *Antimicrob. Agents Chemother.,* 1998, vol. 42, 3097-3102

LISTE DE SEQUENCES

<110> INSTITUT POUR LA RECHERCHE ET LE DEVELOPPEMENT

<120> SOUCHES DE PROTOZOAIRE DE VIRULENCE ATTENUEE ET LEUR UTILISATION

<130> WOB 03 BZ IRD LEIS

<160> 6

<170> PatentIn version 3.1

<210> 1
<211> 24
<212> ADN
<213> Séquence artificielle

<220>
<223> Amorce PCR

<400> 1
gaattcgata tgacagggtc tccg 24

<210> 2
<211> 24
<212> ADN
<213> Séquence artificielle

<220>
<223> Amorce PCR

<400> 2
ctcgagcagt caccatgttg gcag 24

<210> 3
<211> 1122
<212> ADN
<213> Leishmania infantum

<400> 3

```
atgacagcgt ctccgagagc gccacatcag gagcatgtcc tcggagagcc gaccttggaa     60
gggcttgcgc actacatcag ggagaagaat gtgcggcgca ttctcgtgct cgtcggagca    120
ggcgccagtg tagctgccgg catcccagac tttcgctcac ctgacaccgg gatctacgcc    180
aacctcggca agtacaacct cgaagacccg accgatgcct tttcactgac ccttctgcgc    240
gagaagccag agatattcta ctctatcgca cgggagctga acttgtggcc tgggcacttt    300
cagcccaccg cggtacatca cttcatccga ctgttgcaag acgagggccg tctcctgcgc    360
tgctgcacgc agaacattga cggcctggag aaggcagcgg gcgtgtcgcc ggagctcctc    420
gtcgaggcgc atggctcttt cgctgctgcc gcctgcattg aatgccacac accattcagc    480
attgagcaga actacctgga ggcgatgagc ggtacggtct cccgctgctc tacatgcggc    540
ggcattgtga agcccaacgt cgttttcttt ggtgaaaatt tgccggacgc gttcttcgac    600
gcgctgcacc acgacgcccc gatcgcggag ctggtcatca tcatcgggac atcgatgcag    660
gtgcacccgt tcgcgttgct gccgtgcgtc gtgcccaagt cagtcccgcg cgttgtcatg    720
```

```
aaccgtgagc gagttggcgg cctcctcttc cgctttcctg atgacccgct caacaccgtc    780
cacgaggatg cggttgccaa ggagggacgc tcgtcctctt cgcagagtcg ttccccgtcc    840
gcgtcgccac ggcgcgagga gggggaaca gaggacagcc cctcgtcgcc aaacgaggag    900
gtcgaagagg cgtcgacgtc cagctcgagc gacggctacg ggcagtacgg tgactaccac    960
gcccaccccg atgtctgccg ggatgttctc ttccgcggcg actgccagga gaacgtggtg   1020
acgctggcgg agtacctggg tctgagcgag gcgctggcaa agcgcatgcg cttatccgat   1080
gcagcaccag ctactgcaca gagggcgccg aatgagacgt ga                      1122
```

<210> 4
<211> 1146
<212> ADN
<213> Leishmania major

<400> 4

```
atgacagggt ctccgagagc gccgcatcag gaacatgccc tcggagagcc gactgtggaa     60
gggcttgcgc gctacatcag ggagaaggat gtgcggcgca ttctcgtgct cgtcggagca    120
ggcgccagcg tagctgccgg catcccagac tttcgctcat ctgacaccgg gatctacgcc    180
aagctcggca agtacaacct cgacgacccg accgatgcct tttcgctgac tcttctgcgc    240
gagaagccag agatattcta ctctatcgca cgggagctga acttgtggcc tgggcacttt    300
cagcccaccg cggtgcatca cttcatccga ctgttgcaag acgagggccg tcttctgcgc    360
tgctgcacgc agaacattga tggtctggag aaggcagcgg gcgtgtcgcc ggagctcctg    420
gtcgaggcgc atgggtcttt cgctgccgcc gcctgcatcg aatgccacac gcccattcagc   480
attgagcaga actacctgga ggcgatgagc ggcacggtgt cccgctgctc tacatgcggc    540
ggcattgtga agccaaacgt cgttttcttt ggtgaaaatt tgccggacgc gttcttcgac    600
gcgctgcacc acgacgcccc gatcgcggag ctggtcatca tcatcgggac atcgatgcag    660
gtgcacccgt tcgcgttact gccgtgcgtc gtgcccaagt ccatcccgcg cgttctcatg    720
aaccgcgagc gagttggcgg cctcctcttc cgctttcctg atgacccgct cgacaccatc    780
cacgacgatg cggttgccaa ggagggacgc tcgtcctctt cgcagagccg ttccccgtcc    840
gcgtcggcgc ggcgcgagga ggggggacg gaggacggct cctcgtcgcc gaacgaggag     900
gtcgaagacg cgtcgacgtc cagttcgagt gacggctacg gtcagtacgg tgactactac    960
gcccaccccg atgtctgccg ggatgttttc ttccgcggcg actgccagga gaacgtgctg   1020
aagctggccg agtgcctggg cctcagggag gcgctggcca agcgatgcgc ttctccggtg   1080
cggcaccagc tacggcacga aagacgtcga atgagacgtg agtctgaatt gctgccaaca   1140
tggtga                                                              1146
```

<210> 5
<211> 775
<212> ADN
<213> Leishmania amazonensis

<400> 5

```
cccctccaacc tagcaagtac aacctcgacg acccgaccga cgccttttca ctgacccttc     60
tgcgggagag acccgagata ttctactcga tcgcacggga gctgaacttg tggcctgggc     120
actttcagcc caccccggta catcacttca tccgactgtt gcaagatgag ggtcgtctac     180
tgcgctgctg cacgcagaac attgacggcc tggagaaggc cgcgggcgtg tcgccagagc     240
tcctcgtcga ggcacatggg tcttttgctg ccgccgcctg cattgaatgc cacacgccat     300
tcagcattga gcagaactac ctggaggcga tgagcggtac ggtctcccgc tgctctacat     360
gcggcggaat tgtaaaacca aacgttgttt tcttcggtga aaatttgccg gacgctttct     420
tcgacgcgct acaccacgat gccccgatcg cggagctgac tatcatcatc gggacatcga     480
tgcaggtgca cccgttcgcg ctgctgccgt gcgtcgtgcc caagtcagtc ccgcgcgttg     540
tcatgaaccg cgagcgagtt ggtggcctcc tcttccgctt tcctgatgac ccgctgaaca     600
ctgtccacga cgatgcggtt gccaaggagg gtcaatcgtc ttcttcgcag agtcgttccc     660
catccgcgtc ggcgcggtgc gagaagggag gggtagagga cagatcttca tcgccgaagg     720
aggaggtcga cgaagcgtcg acgtccggct cgagcgacgg ctacgggcag tacgg          775
```

<210> 6
<211> 1056
<212> ADN
<213> Trypanosoma brucei

<400> 6

```
atgacagaac cgaagttagc aaccacgcac gtagtgggtg aacccacctt cgaaggactg     60
gcacggttca ttgagcgaaa caacatcacc aaaatatttg ttatggtggg cgcagggata    120
agcgttgcag ctggaatccc cgacttccgc tctccccaca ccggcttgta cgctaaactc    180
agtcgctaca atctcaactc accggaggac gccttctcac tccctctctt gcgtcaacaa    240
ccaagtgtgt tttacaacat tctgatggat atggacctct ggcccgggaa gtattgtcct    300
acgacggttc accactttat cagtctactc gccaagaagg gcatgttatt atgctgttgt    360
acgcagaaca tagacgggtt ggaacgcgcc tgcggaattc cagagtcttt actagttgaa    420
gcccatggtt ccttctcttc cgcatcatgt gttgactgtc acgcgaaata tgacatcaac    480
atcgcgaggg cggagacaag ggctggaaaa gtgcctcatt gcaatcaatg tggtggtata    540
gtgaaacccg acgtggtttt ctttggcgag aatctcccgg aggcgttttt taacgtagcg    600
ggactcattg aggaaacgga attgctgctt attttgggaa cctcacttca agtccaccca    660
tttgccgacc ttgcgctcat ggtgccctct gacgtgccac gagtgttgtt taacttggag    720
cgtgtgggcg ggaggatgtt ccgctttcct acggaccgaa cacccaattt ccgcgccagt    780
tcctatcgtc tcagcactgg aaatggcaat ggcagtaaaa ttagcagtgg ggacagcagc    840
```

```
agcagcagca gcgtcgacgg gtatgaccag tttacgctcg cagagaatga cgagacgggt    900
gtgttgcgtg acattttctt tcctggtgac tgtcaggtgt ctgttcgttc ctttgctcag    960
gcgttgggct tcggagagca gcttgacgcc tctgtacgtg agggaaggga aatatttgag   1020
cgcactcggc gtagggaaaa agtcgttgag ggttaa                             1056
```